# EUROPEAN PATENT SPECIFICATION

(11) **EP 1 055 720 B1**
(45) Date of publication and mention of the grant of the patent: **11.08.2004**
(21) Application number: 00304519.2
(22) Date of filing: 26.05.2000
(51) Int. Cl.: C09K 15/28, C09K 15/30, A61K 7/48

(54) **Compositions for stabilizing oxygen-labile species**
Zusammensetzungen zur Stabilisierung von sauerstoffempfindlichen Verbindungen
Compositions pour la stabilisation de composés sensibles à l'oxygène

(30) Priority: 28.05.1999 US 136442 P; 27.07.1999 US 361425
(43) Date of publication of application: 29.11.2000
(73) Proprietor: JOHNSON & JOHNSON CONSUMER COMPANIES, INC., Skillman, NJ 08558 (US)
(72) Inventor: Kung, John, Somerset, New Jersey 08873 (US); Liu, Jue-Chen, Belle Mead, New Jersey 08502 (US)
(74) Representative: Fisher, Adrian John

(56) References cited:
- EP-A- 0 280 606
- EP-A- 0 349 797
- EP-A- 0 800 825
- WO-A-93/00085
- WO-A-97/21423
- WO-A-97/31620
- DD-A- 150 694
- DE-A- 3 814 806
- FR-A- 2 596 986
- FR-A- 2 641 696
- FR-A- 2 768 623
- DATABASE CHEMABS [Online] CHEMICAL ABSTRACTS SERVICE, COLUMBUS, OHIO, US; STN, CAPLUS accession no. 84:140738, XP002148542 -& CHEMICAL ABSTRACTS, vol. 84, no. 20, 17 May 1976 (1976-05-17) Columbus, Ohio, US; abstract no. 140738, XP002148541 & JP 49 092219 A (S.S. PHARMACEUTICAL CO LTD)
- DATABASE CHEMABS [Online] CHEMICAL ABSTRACTS SERVICE, COLUMBUS, OHIO, US; STN, CAPLUS accession no. 1990:42380, XP002148543 & ACTA FAC. PHARM. UNIV. COMENIAE, vol. 42, 1989, pages 5-19,
- DATABASE CHEMABS [Online] CHEMICAL ABSTRACTS SERVICE, COLUMBUS, OHIO, US; STN, CAPLUS 1971:440469, XP002148544 & JP 46 009358 B (TAKEDA CHEMICAL INDUSTRIES LTD)
- DATABASE CHEMABS [Online] CHEMICAL ABSTRACTS SERVICE, COLUMBUS, OHIO, US; STN, CAPLUS accession no. 1990:164753, XP002148545 & JP 01 256586 A (KOBAYASHI KOSE CO LTD) 13 October 1989 (1989-10-13)
- DATABASE CHEMABS [Online] CHEMICAL ABSTRACTS SERVICE, COLUMBUS, OHIO, US; STN, CAPLUS accession no. 1997:564853, XP002148546 & JP 09 176679 A (OKAWA SHOKUHIN KOGYO KK) 8 July 1997 (1997-07-08)

## Description

### Field of the Invention

This invention relates to compositions and methods for stabilizing oxygen-labile species in compositions. More particularly, it relates to compositions containing oil- and water-soluble oxygen-labile species and stabilizing elements. It also relates to methods of making such compositions and methods of using such compositions.

### Background of the Invention

For many years, it has proven difficult to make stable compositions containing oxygen-labile species. "Oxygen-labile" species are those that are easily oxidized and readily decompose when exposed to the environment. Such oxygen-labile species are, therefore, very difficult to formulate into compositions in combination with other compounds that may accelerate such decomposition or that may be exposed to the environment over time. In recent years, it has become desirable, for example, to include various vitamin compounds in topical skin care compositions in order to nourish or repair the skin. However, those of ordinary skill in the art have found that these vitamin compounds are quite unstable in topical compounds due to the fact that they are easily oxidized and decompose quickly, resulting in loss of efficacy and discoloration of the composition.

Thio compounds such as metabisulfite and sulfite compounds have been known as good water-soluble antioxidants and have been added to compositions to prevent such oxidation problems. However, these compounds may cause sensitization in certain individuals and are not useful for administration to humans. They also possess noxious odors and are unpleasant to use. Japanese Patent Publication No. 53-7488, for example, suggests that combining ascorbic acid with dl-N-acetyl homocysteine thiolactone or N-acetyl-L-cysteine and sulfite in an aqueous solution of ascorbic acid results in a composition that can be stored stably over a long period.

EP 0 349 797 B1 mentions the combination of N-acetylcysteine and ascorbic acid or ascorbate as a stabilizer for the N-acetylcysteine. Slovakian publication, Farm. Obzor-LIV - 1985 p. 513 (Pharmacology Review) mentions the combination of N-acetylcysteine and ascorbic acid at a pH of 6.2. Both N-acetylcysteine and ascorbic acid are water-soluble. However, none of these references indicate that N-acetylcysteine would serve to stabilize an oil-soluble oxygen-labile species.

Thus, it is an object of this invention to provide compositions that contain oxygen-labile species that are stable over long periods of time.

Yet another object of this invention is to provide compositions that contain both oil-soluble and water-soluble oxygen-labile species that are stable over long periods of time.

It is another object of this invention to provide methods of making stable compositions containing oxygen-labile species.

Another object of this invention is to provide methods of using the stable oxygen-labile species-containing compositions of this invention.

Yet another object of this invention is to provide skin care compositions that contain oxygen-labile species such as vitamins and their derivatives for topical use on the skin.

### Summary of the Invention

We have discovered that certain oil-soluble and water-soluble oxygen-labile species may be stabilized in compositions by the addition of stabilizer compounds. Such stabilizer compounds are selected from the following categories:
a) thio-containing compounds, such as sulfites and cysteine derivatives; and
b) glycoproteins, such as lactoferrin.
Oil-soluble oxygen-labile species may include vitamin compounds such as retinoids, choleciferol, vitamin K, tocotrienol and tocopherol derivatives, essential fatty acids and the like. Water-soluble oxygen-labile species may include vitamin compounds such as ascorbic acid and its derivatives, niacin, thiamine, riboflavin, folic acid, pyrodoxine, pantothenic acid, niacinamide, lipoic acid, dihydrolipoic acid, essential amino acids and the like. The oil-soluble oxygen-labile species may be present in the compositions in amounts of from about 0.01 to about 10%. The water-soluble oxygen-labile species may be present in the compositions in amounts of from about 0.01 to about 20%.

### Detailed Description of the Preferred Embodiments

The compositions of this invention contain certain oil-soluble and water-soluble oxygen-labile species which are stabilized in compositions by the addition of stabilizer compounds. Such stabilizer compounds are selected from the following categories:
a) thio-containing compounds, such as sulfites and cysteine derivatives; and
b) lactoferrin.
The stabilizers which are included in the term "thio-containing compounds" are selected from sulfites and metabisulfites, cysteine derivatives and glutathione. More preferably, the thio-containing compounds are cysteine derivatives. Most preferably, the thio-containing compound is N-acetylcysteine. Thio-containing compounds are well-known as stabilizers of water-soluble compounds as they reside in the water phase of compositions. However, they are not known to be capable of stabilizing oil-soluble compounds as they do not reside in the oil phase of compositions. Surprisingly, we have found that compositions containing both water- and oil-soluble oxygen labile species as well as thio-containing compounds are stable over long periods of time. Preferably, the amount of thio-containing compound should range from about 0.001 to about 5% of the weight of the composition. More preferably, there should be from about 0.01 to about 0.5% of the composition by weight.

Furthermore, compositions containing oil-soluble and water-soluble oxygen labile species and stabilizer compounds, selected from the group of glycoproteins are surprisingly stable. The glycoprotein that is useful in the compositions of this invention is lactoferrin. Unexpectedly, such glycoproteins, large molecules that reside in the water phase of compositions, and which are known to be biologically active have proven to be viable as stabilizer compounds in the compositions. Although it is unknown how these proteins serve to stabilize the compositions, it is theorized that the proteins may reside at the oil/water interface of the compositions and are therefore active in both phases. Alternatively, the proteins may somehow adsorb or attract the ions that would otherwise cause oxidation of the oxygen-labile species of the compositions. Preferably, the amount of glycoprotein compound should range from about 0.00001 to about 5% of the weight of the composition. More preferably, there should be from about 0.01 to about 1% of the composition by weight.

The glycoprotein useful in the compositions of this invention is lactoferrin, a milk-derived protein that chelates iron and has a molecular weight of 80,000 daltons. Although lactoferrin is known as an anti-free radical compound in biological systems, it has heretofore been unknown for use in formulations. Whether an antioxidant or chelator which is active in a biological system will be active in a composition is completely unpredictable.

The oxygen-labile species exist in the compositions of this invention as a combination. Vitamins A, C and E are present in the composition with both a thio-containing compound and lactoferrin. Surprisingly, both the water-soluble and oil-soluble oxygen-labile species are stable over a long period of time. One of ordinary skill in the art would expect that vitamins A and C together would not be stable as Vitamin C tends to sacrifice itself to stabilize Vitamin A. Compositions of this invention containing both Vitamins A and E together, we have found, preferably contain at least about 0.0001% of Vitamin C for ensuring stability of all materials.

Actually, compositions of this invention comprise a retinoid, ascorbic acid or a derivative thereof, tocopherol or a derivative thereof, a thio-containing compound selected from sulfites, metabisulfites, cysteine derivatives and glutathione, and lactoferrin.

The compositions of this invention may be utilized in dosage forms suitable for cosmetic or pharmaceutical use. For example, the compositions of this invention may be made in the forms of emulsions, creams, lotions, gels, essences, milks, toners, hydroalcoholic solutions, multivesicular systems, suspensions, patches, masks, sticks, and other dosage forms suitable for therapeutic use, including oral administration forms.

The compositions of this invention may be made using conventional formulation technology. For example, in a standard oil-in-water emulsion, the starting water phase should be purged with either nitrogen or argon to displace any residual oxygen. Alternatively, the water phase may be heated to 80°C and held at that temperature at least about ten minutes to reduce oxygen solubility. A conventional oil phase should be made and the oil phase poured into the water phase. After phasing, the formulation should be blanketed with an inert gas such as nitrogen or argon and the formulation permitted to cool to room temperature. As the temperature reaches 45° C, the stabilizer compound should be added to the formulation. The stabilizer compound should be mixed into the formulation for about ten minutes, after which the oxygen-labile species may be introduced into the formulation. Neutralization to the appropriate pH may be made prior to or subsequent to the addition of the oxygen-labile species, depending upon the oxygen-labile species utilized. For example, neutralization is preferred subsequent to adding ascorbic acid, but should be accomplished prior to adding retinol. The formulation resulting from this process should be packaged in an oxygen-impermeable package, such as an aluminum tube.

The pH of the formulations of this invention is preferably in a range that is suitable for a particular oxygen-labile species used in the compositions. The pH should reflect as closely as possible the physiological pH of the skin without compromising the chemical stability of the oxygen-labile species. For example, the preferred pH environment for ascorbic acid should about 5.5 and above. The preferred pH for retinol should be about 5.5 and above. Preferably, the pH should be between about 5.5 and about 9. pH greater than about 10 may result in irritation to the skin when applied topically.

During manufacture of the compositions of this invention, oxygen exposure should be minimized as much as possible so as to reduce the possibility of oxidizing the oxygen-labile species and preserve its stability. Therefore, to the extent possible, all oxygen in the manufacturing system should be displaced with nitrogen or argon gas, as set forth, for example, in U.S. Patent No. 5,559,149.

The compositions of this invention may be used in therapeutic situations wherever the oxygen-labile ingredients are therapeutically active. For example, ascorbic acid can be used for collagen synthesis, elastin synthesis or skin depigmentation and other known uses. Tocopherol, for example, may be useful in free radical scavenging internally or topically. The compositions of this invention may be applied topically to the skin on a daily or more or less frequent basis. The compositions of this invention deliver therapeutic quantities of oxygen-labile species to the skin.

Other emollients and surface active agents have been incorporated in the emulsions, including glycerol trioleate, acetylated sucrose distearate, sorbitan trioleate, polyoxyethylene (1) monostearate, glycerol monooleate, sucrose distearate, polyethylene glycol (50) monostearate, octylphenoxypoly (ethyleneoxy) ethanol, decaglycerin penta-isostearate, sorbitan sesquioleate, hydroxylated lanolin, lanolin, triglyceryl diisostearate, polyoxyethylene (2) oleyl ether, calcium stearoyl-2-lactylate, methyl glucoside sesquistearate, sorbitan monopalmitate, methoxy polyethylene glycol-22/dodecyl glycol copolymer (Elfacos E200), polyethylene glycol-45/dodecyl glycol copolymer (Elfacos ST9), polyethylene glycol 400 distearate, and lanolin derived sterol extracts, glycol stearate and glycerol stearate; alcohols, such as cetyl alcohol and lanolin alcohol; myristates, such as isopropyl myristate; cetyl palmitate; cholesterol; stearic acid; propylene glycol; glycerine, sorbitol and the like.

The composition of this invention can contain additives, as required, such as a humectant, an antioxidant, a preservative, a flavor, fragrances, a surface active agent, a binder, and the like, as well as skin protectant agents, therapeutic agents and "cosmeceuticals".

Examples of the preservatives include salicylic acid, chlorhexidine hydrochloride, phenoxyethanol, sodium benzoate, methyl para-hydroxybenzoate, ethyl para-hydroxybenzoate, propyl para-hydroxybenzoate, butyl para-hydroxybenzoate and the like.

Examples of the flavor and fragrance include menthol, anethole, carvone, eugenol, limonene, ocimene, n-decylalcohol, citronellol, a-terpineol, methyl salicylate, methyl acetate, citronellyl acetate, cineole, linalool, ethyl linalool, vanillin, thymol, spearmint oil, peppermint oil, lemon oil, orange oil, sage oil, rosemary oil, cinnamon oil, pimento oil, cinnamon leaf oil, perilla oil, wintergreen oil, clove oil, eucalyptus oil and the like.

Examples of surface active agents include sodium alkyl sulfates, e.g., sodium lauryl sulfate and sodium myristyl sulfate, sodium N-acyl sarcosinates, e.g., sodium N-lauroyl sarcosinate and sodium N-myristoyl sarcosinate, sodium dodecylbenzenesulfonate, sodium hydrogenated coconut fatty acid monoglyceride sulfate, sodium lauryl sulfoacetate and N-acyl glutamates, e.g., N-palmitoyl glutamate, N-methylacyltaurin sodium salt, N-methylacylalanine sodium salt, sodium a-olefin sulfonate and sodium dioctylsulfosuccinate; N-alkylaminoglycerols, e.g., N-lauryldiaminoethylglycerol and N-myristyldiaminoethylglycerol, N-alkyl-N-carboxymethylammonium betaine and sodium 2-alkyl-1-hydroxyethylimidazoline betaine; polyoxyethylenealkyl ether, polyoxyethylenealkylaryl ether, polyoxyethylenelanolin alcohol, polyoxyethyleneglyceryl monoaliphatic acid ester, polyoxyethylenesorbitol aliphatic acid ester, polyoxyethylene aliphatic acid ester, higher aliphatic acid glycerol ester, sorbitan aliphatic acid ester, Pluronic type surface active agent, and polyoxyethylenesorbitan aliphatic acid esters such as polyoxyethylenesorbitan monooleate and polyoxyethylenesorbitan monolaurate. Emulsifier-type surfactants known to those of skill in the art should be used in the compositions of this invention.

Examples of the binder or thickener include cellulose derivatives such as alkali metal salts of carboxymethylcellulose, methyl cellulose, hydroxyethyl cellulose and sodium carboxymethylhydroxyethyl cellulose, alkali metal alginates such as sodium alginate, propylene glycol alginate, gums such as carrageenan, xanthan gum, tragacanth gum, caraya gum and gum arabic, and synthetic binders such as polyvinyl alcohol, polysodium acrylate and polyvinyl pyrrolidone.

Thickeners such as natural gums and synthetic polymers, as well as preservatives such as methylparaben, butyl paraben, propylparaben and phenoxyethanol, coloring agents and fragrances also are commonly included in such compositions.

Other active ingredients such as sunscreen materials and antimicrobial materials may be utilized in the compositions of the present invention provided that they are physically and chemically compatible with the other components of the compositions. For example, moisturizing agents such as propylene glycol, allantoin, acetamine MEA, oat protein and hyaluronic acid and other humectants may be added to the retinoid-containing formulations of this invention in order to provide moisturizing activity in conjunction with the retinoid-related activity of the products. Other proteins and amino acids may also be incorporated. Sunscreens may include organic or inorganic sunscreens, such as octylmethoxycinnamate and other cinnamate compounds, titanium dioxide and zinc oxide and the like.

Other ingredients may include agents which assist in protecting the skin from aging, such as sunscreens, anti-oxidant vitamins such as ascorbic acid, vitamin B, biotin, pantothenic acid, vitamin D, vitamin E and vitamin C. Yeast extract, gingko biloba, bisabolol, panthenol, alpha hydroxy acids and oligosaccharides such as melibiose are among other ingredients which assist in preventing aging of the skin by such means as irritation mitigation, oxidation mitigation, healing, affecting retinoid metabolism and inhibiting the production of elastase.

Skin color evening ingredients and depigmentation agents may also be effective in the products of this invention. Such ingredients may include hydroquinone, licorice extract, kojic acid, gatuline A (pilewort extract), micromerol (butylene glycol and apple extract), glutathione, arbutin, placenta extract, ascorbic acid, magnesium-L-ascorbyl-2-phosphate and the like.

Compositions which assist in the reduction of lines and wrinkles may also be added to the compositions of this invention. For example, alpha hydroxy acids, hyaluronic acid, Gatuline R (fagus silvitica extract), pigments and scattering aids such as mica, zinc oxide and titanium dioxide may be used in the compositions of this invention in this capacity. Various natural extracts such as tannins, flavenoids, saponins and the like may also be added.

Anti-inflammatory agents may also be used in the compositions of this invention. Not only should these agents assist in mitigating irritation, they may assist the retinoids in treating wrinkles and lines in the skin. Steroidal anti-inflammatory agents, including but not limited to, corticosteroids such as hydrocortisone, hydroxyltriamcinolone, alpha-methyl dexamethasone, dexamethasone-phosphate, beclomethasone dipropionate, clobetasol valerate, desonide, desoxycorticosterone acetate, dexamethasone, dichlorisone, deflorasonediacetate, diflucortolone valerate, fluadronolone, fluclarolone acetonide, fludrocortisone, flumethasone pivalate, fluosinolone acetonide, fluocionide, flucortine butylester, fluocortolone, flupredidene (flupredylidene) acetate, flurandronolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone and its esters, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone and mixtures thereof may be used. Preferably, hydrocortisone may be used.

Nonsteroidal anti-inflammatory agents may also be employed in the compositions of this invention, such as salicylates (including alkyl and aryl esters of salicylic acid), acetic acid derivatives (including arylacetic acid and its derivatives), fenamates, propionic acid derivatives and pyrazoles or mixtures thereof. Other synthetic and natural anti-inflammatory agents may also be used.

Additional active ingredients having topical activity may be utilized in the compositions of this invention. Azole-type anti-fungal and anti-bacterial agents may be employed in the compositions of this invention in their base form. For example, ketoconazole, miconazole, itraconazole, metronidazole, elubiol, and like related imidazole antifungals and antibacterials are useful in the topical formulations of this invention.

The advantages of the invention and specific embodiments of the skin care compositions prepared in accordance with the present invention are illustrated by the following examples.

### Example 1: An Emulsion containing A Hydrophilic Oxygen-Labile Species (Ascorbic Acid)

A composition was made by combining the following ingredients in a water phase:

| Ingredients | % w/w |
|---|---|
| Water | q.s. to 100% |
| Disodium EDTA | 0.10% |
| Glycerin | 5.00% |
| Phenoxyethanol | 0.73% |
| Methylparaben | 0.20% |
| Propylparaben | 0.07% |
| Hydroxyethyl cellulose | 0.3% |
| Xanthan Gum | 0.50% |

A batch of 1 kilogram was made according to the following process. The weight of the beaker was recorded, and the water then added. Because this process requires boiling, an additional 50 grams of water was added to the beaker to counter the effect of evaporation. The beaker was covered with aluminum foil and beginning boiling the water. The water was permitted to boil for at least five minutes. The heat was turned off, and the water cooled to 80°C. The water phase was kept heated at least for ten minutes. The disodium EDTA was added and the water phase mixed until the EDTA was fully dissolved and clear. The xanthan gum and hydroxyethyl cellulose were slurried into the glycerin in a side container. The slurry was poured into the water phase. Tthe preservatives were added (i.e., the phenoxyethanol, methyl paraben and propyl paraben). The water phase was held at 80°.

Separately, in another container, an oil phase was created using the following ingredients:

| **Ingredients** | **% w/w** |
|---|---|
| Butyl hyroxytoluene | 0.10% |
| Glyceryl monostearate & Peg-100 stearate | 5.00% |
| Cetyl palmitate | 1.00% |
| Cetyl alcohol | 1.00% |
| C12-15 alkyl benzoate | 4.00% |
| White petrolatum | 1.50% |
| Butyl Methoxydibenzoyl methane | 1.00% |
| Octyl methoxycinnamate | 7.50% |

All of the above named oil phase ingredients were combined in a beaker, and heated to 80°C. The oil phase was mixed homogeneously. When both phases were at 80°C, the oil was phased into the water phase. The batch was cooled to room temperature. When at 45°C, the post-additions were added to the batch.

| **Ingredients** | **%w/w** |
|---|---|
| Ascorbic acid | 5.00% |
| N-acetyl cysteine | 0.10% |
| Ethanol | 2.78% |
| NaOH (20%) | q.s. to desired pH. |

The stabilizing compound, in this case the N-acetyl cysteine was added first, and allowed to mix for at least ten minutes. After the requisite mixing, the ascorbic acid was added. The sodium hydroxide was then added to neutralize the batch. The mixer speed was slowed down when the neutralization solution was added to minimize any whipping of air into the beaker. Finally, the ethanol was added , and water added to the batch q.s. The product was filled in aluminum tubes. The compositions may also be placed in any other suitable oxygen impermeable package.

### Example 6:

The stability of different formulations containing retinol, ascorbic acid and/or tocopherol as set forth in Examples 7 and 8 below were monitored in terms of appearance and HPLC assay. The degraded products of retinol, ascorbic acid or tocopherol are yellow or brownish in color. The freshly prepared samples were white creams. Discoloration to yellow or brown indicates the instability.

| **Effect of 1% Iniferine on Retinol, Ascorbic acid and Tocopherol stability (no BHT, no EDTA)** | | | | | |
|---|---|---|---|---|---|
| Example # | Active | Assay (4°C, 4 weeks) | Color (4°C, 4 weeks) | Assay (40°C, 4 weeks) | Color (40°C, 4 weeks) |
| Example 7 | Retinol | 0.168 | White (no discoloration) | 0.122 (72.6%) | Yellowish (discolored) |
| | Ascorbic acid | 4.8 | | 4.68 (97.5%) | |
| | Tocopherol | 0.95 | | 0.64 (67.4%) | |
| Example 8 | Retinol | 0.174 | White (no discoloration) | 0.165 (94.8%) | White (no discoloration) |
| | Ascorbic acid | 4.81 | | 4.63 (96.5%) | |
| | Tocopherol | 1.01 | | 0.98 (97.0%) | |

If ascorbic acid is removed from the formulations, as set forth below, the formulations are not as stable, even with additional BHT and EDTA. However, formulations containing ascorbic acid and tocopherol were found to be stable. Thus, ascorbic acid assists in stabilizing tocopherol.

| **Effect of 5% Ascorbic acid on Retinol and Tocopherol stability** | | | | |
|---|---|---|---|---|
| Example # | active | Target value | 25°C, 1 week | Color |
| Example 9 | Retinol | 0.1725 | 0.1695 | White (no discoloration) |
| | Ascorbic Acid | 5.00 | 4.96 | |
| | Tocopherol | 1.00 | 0.96 | |
| Example 10 | Retinol | 0.1725 | 0.15 | Bright yellow (discolored) |
| | Tocopherol | 1.00 | 0.89 | |

Due to the immediate loss of retinol and tocopherol and significant discoloration, no further stability was conducted on Example 10. The addition of N-acetylcysteine slightly enhances the stability of retinol and tocopherol.

| **Effect of Ascorbic concentration on Ascorbic acid, Retinol and Tocopherol Stability (compositions referred to in Example 9)** | | | |
|---|---|---|---|
| Initial Ascorbic concentration | active | 8 weeks @ 4°C | 8 weeks @ 40°C |
| 0.1% | Retinol | 0.1655 (100%) | 0.1600 (96.7%) |
| | Ascorbic Acid | 0.05 (100%) | 0.04 (80%) |
| | Tocopherol | 0.98 (100%) | 1.00 (100%) |
| 1% | Retinol | 0.1582 (100%) | 0.1603 (100%) |
| | Ascorbic Acid | 0.89 (100%) | 0.80 (89.9%) |
| | Tocopherol | 0.99 (100%) | 0.99 (100%) |
| 10% | Retinol | 0.1657 (100%) | 0.1664 (100%) |
| | Ascorbic acid | 9.80 (100%) | 9.90 (100%) |
| | Tocopherol | 1.10 (100%) | 1.12 (100%) |

If we refer to the stability of the compositions at 8 weeks at 40°C as the initial, all A (retinol), C (ascorbic acid), E (tocopherol) samples are stable. The data also suggests that it is important to control the manufacturing process for low concentration ascorbic acid to minimize any loss in stability.

### Example 7:

A formulation was made containing Vitamins A, C, E and iniferine. It did not include BHT, an antioxidant or disodium EDTA, a chelating agent.

| **Chemical Name** | **% wt/wt** |
|---|---|
| *Water Phase* | |
| Water | 65.15% |
| Disodium EDTA | 0.00% |
| Glycerin | 5.00% |
| Preservative | 0.73% |
| Preservative | 0.35% |
| Preservative | 0.17% |
| Hydroxyethylcellulose | 0.30% |
| Xanthan Gum | 0.50% |

| *Oil Phase* | |
|---|---|
| Butylhydroxytoluene | 0.00% |
| Glyceryl Monostearate & PEG-100 | 5.00% |
| Cetyl Palmitate | 1.00% |
| Cetyl Alcohol | 1.00% |
| Stearyl Alcohol | 0.50% |
| C_{12-C15} Alkyl Benzoate | 4.00% |
| White Petrolatum | 1.50% |

| *Post - Additions* | |
|---|---|
| Ascorbic Acid | 5.00% |
| Tocopherol | 1.00% |
| Retinol | 0.40% |
| Lactoferrin; thioxanthine; uric acid | 0.00% |
| Ethanol | 2.78% |
| NaOH (20%) | 5.62% |

### Example 8:

A formulation was made containing Vitamins A, C, E and iniferine. It included BHT, an antioxidant and disodium EDTA, a chelating agent.

| **Chemical Name** | **% wt/wt** |
|---|---|
| *Water Phase* | |
| Water | 64.15% |
| Disodium EDTA | 0.00% |
| Glycerin | 5.00% |
| Phenoxyethanol | 0.73% |
| Methyl paraben | 0.35% |
| Propyl paraben | 0.17% |
| Hydroxyethylcellulose | 0.30% |
| Xanthan Gum | 0.50% |

| *Oil Phase* | |
|---|---|
| Butylhydroxytoluene | 0.00% |
| Glyceryl Monostearate & PEG-100 | 5.00% |
| Cetyl Palmitate | 1.00% |
| Cetyl Alcohol | 1.00% |
| Stearyl Alcohol | 0.50% |
| C_{12-C15} Alkyl Benzoate | 4.00% |
| White Petrolatum | 1.50% |

| *Post - Additions* | |
|---|---|
| Ascorbic Acid | 5.00% |
| Tocopherol | 1.00% |
| Retinol | 0.40% |
| Lactoferrin; thioxanthine; uric acid | 1.00% |
| Ethanol | 2.78% |
| NaOH (20%) | 5.62% |

### Example 9:

Another formulation was made containing Vitamins A, C and E but did not include sunscreens. The composition was made in accordance with the procedure set forth in Example 1.

| **Chemical Name** | **% wt/wt** |
|---|---|
| *Water Phase* | |
| Water | 63.95% |
| Disodium EDTA | 0.10% |
| Glycerin | 5.00% |
| Phenoxyethanol | 0.73% |
| Methyl paraben | 0.35% |
| Propyl paraben | 0.17% |
| Hydroxyethylcellulose | 0.30% |
| Xanthan Gum | 0.50% |

| *Oil Phase* | |
|---|---|
| Butylhydroxytoluene | 0.10% |
| Glyceryl Monostearate & PEG-100 | 5.00% |
| Cetyl Palmitate | 1.00% |
| Cetyl Alcohol | 1.00% |
| Stearyl Alcohol | 0.50% |
| C_{12-C15} Alkyl Benzoate | 4.00% |
| White Petrolatum | 1.50% |

| *Post-Additions* | |
|---|---|
| Ascorbic Acid | 5.00% |
| Tocopherol | 1.00% |
| Retinol | 0.40% |
| Lactoferrin; thioxanthine; uric acid | 1.00% |
| Ethanol | 2.78% |
| NaOH (20%) | 5.62% |

Another 3 formulations containing 0.1%, 1% and 10% ascorbic acid respectively were made in accordance with example 9, however, 1% lactoferin/thioxanthine/uric acid was replaced with 1% lactoferrin.

### Example 10:

Yet another formulation was made in accordance with the method set forth in Example 1. This composition contained Vitamins A and E and iniferine.

| **Chemical Name** | **% wt/wt** |
|---|---|
| Water | 68.95% |
| Disodiurn EDTA | 0.10% |
| Glycerin | 5.00% |
| Phenoxyethanol | 0.73% |
| Methyl paraben | 0.35% |
| Propyl paraben | 0.17% |
| Hydroxyethylcellulose | 0.30% |
| Xanthan Gum | 0.50% |
| | |
| Butylhydroxytoluene | 0.10% |
| Glyceryl Monostearate & PEG-100 | 5.00% |
| Cetyl Palmitate | 1.00% |
| Cetyl Alcohol | 1.00% |
| Stearyl Alcohol | 0.50% |
| C_{12-C15} Alkyl Benzoate | 4.00% |
| White Petrolatum | 1.50% |
| | |
| Tocopherol | 1.00% |
| Retinol | 0.40% |
| Lactoferrin; thioxanthine; uric acid | 1.00% |
| Ethanol | 2.78% |
| NaOH (20%) | 5.62% |

### Example 12:

A composition was made containing Vitamins A and C with Iniferine alone without N-acetyl cysteine. After 13 weeks exposure to 40°C, 95% of the Vitamin C was retained and there was no loss in A.

| **Chemical Name** | **% wt/wt** |
|---|---|
| Water Phase | |
| Water | 73.96% |
| Disodium EDTA | 0.20% |
| Phenoxyethanol | 0.73% |
| Methyl paraben | 0.20% |
| Propyl paraben | 0.07% |
| Hydroxyethylcellulose | 1.00% |

| Oil Phase | |
|---|---|
| Butylhydroxytoluene | 0.10% |
| GMS | 2.00% |
| Cetearyl Glucoside | 3.00% |
| C12-15 alkyl benzoate | 2.00% |
| Avobenzone | 2.00% |
| Octyl methoxycinnamate | 4.00% |
| Ascorbyl Palmitate | 0.50% |

| Post - Additions | |
|---|---|
| ascorbic acid | 2.00% |
| n-acetyl cysteine | 0.00% |
| Retinol 50c | 0.27% |
| uric acid | 1.00% |
| isoparaffin; laureth-7; polyacrylamide | 1.50% |
| NaOH | 5.47% |

### Example 13:

The following composition contained only Vitamin C with N-acetyl cysteine. It was made in accordance with the procedure set forth in Example 1 except that the composition was boiled rather than purged with inert gas to remove oxygen.

| **Chemical Name** | **% wt/wt** |
|---|---|
| *Water Phase* | |
| Water | 64.92% |
| Disodium EDTA | 0.10% |
| Glycerin | 5.00% |
| Phenoxyethanol | 0.73% |
| Methyl paraben | 0.20% |
| Propyl paraben | 0.07% |
| Hydroxyethylcellulose | 0.30% |
| Xanthan Gum | 0.50% |

| *Oil Phase* | |
|---|---|
| Butylhydroxytoluene | 0.10% |
| Glyceryl Monostearate & PEG-100 | 5.00% |
| Cetyl Palmitate | 1.00% |
| Cetyl Alcohol | 1.00% |
| Stearyl Alcohol | 0.50% |
| C12-C15 Alkyl Benzoate | 4.00% |
| White Petrolatum | 1.50% |
| Avobenzone | 1.00% |
| Octyl methoxycinnamate | 7.50% |

| *Post - Additions* | |
|---|---|
| Ascorbic Acid | 2.00% |
| ethanol | 2.78% |
| n-Acetyl Cysteine | 0.10% |
| NaOH (10%) | 1.70% |

After exposure to 50°C for 13 weeks, 96% of the Vitamin C remained in this composition.

### Example 14:

A composition in accordance with this invention was made using the procedure set forth in Example 1. This composition contained Vitamins A and C as well as Iniferine and N-acetyl cysteine. After 13 weeks incubation at 40°C, 90% C and 96% A remained in the composition.

| **Chemical Name** | **% wt/wt** |
|---|---|
| *Water Phase* | |
| Water | 51.27% |
| Disodium EDTA | 0.10% |
| Glycerin | 5.00% |
| Phenoxyethanol | 0.73% |
| Methyl paraben | 0.35% |
| Propyl paraben | 0.17% |
| Hydroxyethylcellulose | 0.15% |
| Xanthan Gum | 0.50% |

| *Oil Phase* | |
|---|---|
| Butylhydroxytoluene | 0.10% |
| Octyl methoxycinnamate | 7.50% |
| Avobenzone | 3.00% |
| Glyceryl Monostearate & PEG-100 Stearate | 5.00% |
| Cetyl Palmitate | 1.00% |
| Cetyl Alcohol | 1.00% |
| Stearyl Alcohol | 0.50% |
| C12-C15 Alkyl Benzoates | 4.00% |
| White Petrolatum | 1.50% |

| *Post - Additions* | |
|---|---|
| Ascorbic Acid | 5.00% |
| Tocopherol | 0.05% |
| Retinol | 0.25% |
| Lactoferrin; thioxanthine; uric acid | 1.00% |
| n-acetyl cysteine | 0.01% |
| Ethanol | 2.78% |
| NaOH (20%) | 9.04% |

### Example 15:

A composition in accordance with this invention was made using the procedure set forth in Example 1. This composition contained Vitamins A, C and E as well as Iniferine and N-acetyl cysteine. After 11.5 weeks incubation at 40°C, 92% of the Vitamin A, 99% of the Vitamin C and 97% of the Vitamin E remained in the composition.

| **Chemical Name** | **% wt/wt** |
|---|---|
| *Water Phase* | |
| Water | 53.45% |
| Disodium EDTA | 0.10% |
| Glycerin | 5.00% |
| Phenoxyethanol | 0.73% |
| Methyl paraben | 0.35% |
| Propyl paraben | 0.17% |
| Hydroxyethylcellulose | 0.30% |
| Xanthan Gum | 0.50% |

| *Oil Phase* | |
|---|---|
| Butylhydroxytoluene | 0.10% |
| Octyl methoxycinnamate | 7.50% |
| Avobenzone | 3.00% |
| Glyceryl Monostearate & PEG-100 | 5.00% |
| Octyl Hydroxstearate | 2.00% |
| Cetyl Alcohol | 2.00% |
| C12-C15 Alkyl Benzoate | 5.00% |

| *Post - Additions* | |
|---|---|
| Ascorbic Acid | 2.00% |
| Tocopherol | 1.00% |
| Retinol | 0.25% |
| Lactoferrin; thioxanthine; uric acid | 1.00% |
| n-acetyl cysteine | 0.01% |
| Cyclomethicone | 1.50% |
| NaOH (10%) | |

## Claims

1. A composition comprising a retinoid, ascorbic acid or a derivative thereof, tocopherol or a derivative thereof, a thio-containing compound selected from sulfites, metabisulfites, cysteine derivatives and glutathione, and lactoferrin.

2. A composition according to claim 1, further comprising a humectant, a further antioxidant, a preservative, a fragrance, a surface active agent, a binder or a skin protectant agent.

## Patentansprüche

1. Zusammensetzung umfassend ein Retinoid, Ascorbinsäure oder ein Derivat davon, Tocopherol oder ein Derivat davon, eine Thio-enthaltende Verbindung ausgewählt aus Sulfiten, Metabisulfiten, Cysteinderivaten und Gluthathion und Lactoferrin.

2. Zusammensetzung nach Anspruch 1, des weiteren umfassend ein Befeuchtungsmittel, ein weiteres Antioxidationsmittel, ein Konservierungsmittel, einen Duftstoff, eine oberflächenaktive Substanz, ein Bindungsmittel oder ein Haut-schützendes Mittel.

## Revendications

1. Composition comprenant un rétinoïde, l'acide ascorbique ou un de ses dérivés, le tocophérol ou un de ses dérivés, un composé contenant un thio choisi parmi les sulfites, les métabisulfites, les dérivés de la cystéine et la glutathione, et la lactoferrine.

2. Composition selon la revendication 1, comprenant en outre un humectant, un autre antioxydant, un conservateur, une fragrance, un agent tensioactif, un liant ou un agent protégeant la peau.
